Europäisches Patentamt

European Patent Office

Office européen des brevets

(11) Numéro de publication : **0 306 445 B1**

(12) **FASCICULE DE BREVET EUROPEEN**

(45) Date de publication du fascicule du brevet :
**12.08.92 Bulletin 92/33**

(51) Int. Cl.⁵ : **A61B 1/12, A61B 17/32**

(21) Numéro de dépôt : **88810357.9**

(22) Date de dépôt : **02.06.88**

(54) Circuit électrique de commande d'un dispositif pour faire circuler un liquide physiologique à travers une cavité du corps.

(30) Priorité : **26.08.87 US 89740**

(43) Date de publication de la demande :
**08.03.89 Bulletin 89/10**

(45) Mention de la délivrance du brevet :
**12.08.92 Bulletin 92/33**

(84) Etats contractants désignés :
**BE CH DE ES FR GB IT LI NL SE**

(56) Documents cités :
**WO-A-86/01390**
**WO-A-87/00759**
**GB-A- 2 093 353**
**US-A- 3 812 855**

(73) Titulaire : **ORTHOCONCEPT S.A.**
**65, rue de la Prulay**
**CH-1217 Meyrin (CH)**

(72) Inventeur : **Mathies, Burkhard**
**Châtel-Dessus**
**CH-1261 Givrins (CH)**
Inventeur : **Misse, Didier**
**Route de Ferney 208A**
**CH-1218 Grand-Saconnex (CH)**
Inventeur : **Guignard, Claude**
**Le Vezely Sergy Gare**
**F-01630 St-Genis - Pouilly (FR)**
Inventeur : **Pidoux, Paul**
**CH-1261 Bassins (CH)**

(74) Mandataire : **Dousse, Blasco et al**
**7, route de Drize**
**CH-1227 Carouge/Genève (CH)**

EP 0 306 445 B1

Jouve, 18, rue Saint-Denis, 75001 PARIS

**Description**

La présente invention se rapporte à un circuit électrique de commande d'un dispositif pour faire circuler un liquide physiologique à débit et pression déterminés à travers une cavité du corps, en vue d'une résection et/ou d'un examen endoscopique, dans lequel, d'une part, un conduit d'alimentation est destiné à relier une source de ce liquide à ladite cavité par l'intermédiaire d'une pompe d'alimentation et d'un capteur de pression associé à un circuit d'asservissement de cette pompe en fonction de la pression mesurée par le capteur et, d'autre part, au moins un conduit de sortie destiné à évacuer le liquide de cette cavité par l'intermédiaire d'une source d'aspiration commandée par un circuit de contrôle, ce circuit de contrôle comprenant des éléments de consigne pour fixer des valeurs respectives du débit de cette source d'aspiration et des moyens pour commuter le circuit de contrôle de cette source sur l'un ou l'autre de ces éléments de consigne.

Un dispositif similaire est décrit dans le WO 86/01390 pour l'arthroscopie des articulations, notamment du genou. A cet effet, une circulation d'eau physiologique est établie dans l'articulation et l'image interne de cette articulation est transmise à un écran vidéo par un réseau de fibres optiques. Cette technique est utilisée aussi bien pour effectuer un examen du ménisque, des ligaments croisés, de la synoviale, du cartilage de la rotule du fémur et du tibia, que pour opérer certaines lésions, telles qu'une rupture méniscale, un repli synovial, un fragment osseux détaché, ou pour effectuer un lavage de l'articulation.

Il est utile qu'un tel dispositif permette non seulement de contrôler la pression de l'eau physiologique dans l'articulation mais encore d'avoir un contrôle sur le débit. En effet dans le cas d'une simple observation, le débit n'est pas critique. Toutefois, dès qu'une résection endoscopique est pratiquée, il devient nécessaire de pouvoir augmenter le débit voire même de faire passe ce débit à travers un conduit spécifique qui peut être amené par le chirurgien à un endroit précis où il désire évacuer certaines impuretés.

Ces dispositifs pour faire circuler de l'eau physiologique comportent un conduit d'entrée et généralement deux conduits de sortie, une sortie générale que l'on implante de préférence au-dessus de l'articulation et une sortie auxiliaire spécifique qui s'effectue notamment à travers un outil de coupe rotatif comprenant un tube externe relié au conduit d'évacuation, présentant une lumière latérale au voisinage de son extrémité et dans lequel tourne un organe de coupe qui coupe et aspire dans le conduit d'évacuation les tissus malades coupés par le chirurgien. Une commutation permet de relier le conduit d'évacuation à travers l'une ou l'autre de ces sorties. La sortie spécifique ou auxiliaire qui passe à travers l'outil de coupe peut également être utilisée indépendemment de cet outil dans le seul but d'effectuer une aspiration localisée d'un fragment de tissu ou d'une autre substance. Dans ces conditions, l'outil de coupe étant mû par un moteur électrique il est utile d'avoir la possibilité d'utiliser l'aspiration auxiliaire seule ou en combinaison avec l'outil de coupe.

Ces diverses possibilités doivent cependant pouvoir être réalisées avec un maximum de facilité et d'autonomie par le chirurgien, tout en lui laissant les mains libres pour effectuer son intervention. Il est évident que les commandes par pédale sont utilisables pour autant que leur actionnement soit suffisamment aisé à manoeuvrer.

Le but de la présente invention est précisément d'apporter des améliorations aux dispositifs de ce genre qui donnent au chirurgien un maximum d'autonomie dans la commande et une diversité de fonctions qui assure au dispositif une souplesse qui lui permette de s'adapter à l'ensemble des situations susmentionnées.

A cet effet, cette invention a pour objet un circuit électrique de commande d'un dispositif pour faire circuler un liquide physiologique à débit et pression déterminés à travers une cavité du corps selon la revendication 1.

Le dessin annexé illustre schématiquement et à titre d'exemple, une forme d'exécution du dispositif et de son circuit de commande objet de la présente invention.

La figure 1 est un schéma du dispositif.

Les figures 2 et 3 sont des schémas blocs du circuit électrique de commande.

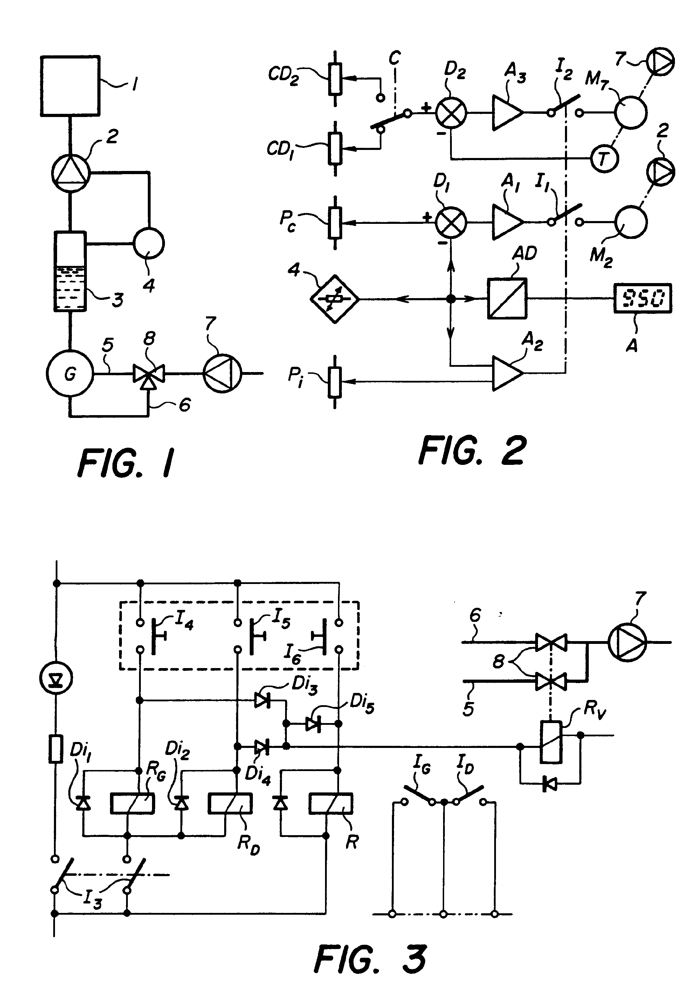

La figure 1 représente une source d'eau physiologique 1, une pompe d'alimentation 2 dont l'entrée est reliée à la source 1 et dont la sortie est reliée à un réservoir intermédiaire 3. Un capteur de pression 4 mesure la pression régnant dans ce réservoir 3 et sert à commander la pompe 2 comme on l'expliquera par la suite. La sortie du réservoir 3 est reliée à l'articulation G.

La sortie de l'articulation G est reliée par deux conduits 5 et 6 à une pompe volumétrique d'aspiration 7, ces conduits étant mis sélectivement en communication avec l'entrée de cette pompe par une électrovanne à trois voies 8. Le conduit 5 correspond au conduit d'évacuation normal qui est généralement implanté au-dessus de l'articulation. Le conduit 6 est un conduit auxiliaire. Il est implanté en face de l'articulation et sert notamment à faire passer l'aspiration à travers un organe de coupe utilisé pour la résection endoscopique, ou comme canal d'aspiration spécifique de déchets de tissus localisés par exemple.

Dans le schéma bloc de la figure 2 on retrouve les deux pompes 2 et 7 entraînées chacune par un moteur respectif $M_2$ et $M_7$. Le moteur $M_2$ est relié à la sortie d'un amplificateur $A_1$ par l'intermédiaire d'un interrupteur $I_1$. L'entrée de cet amplificateur est connectée à un différentiateur $D_1$ qui établit l'écart entre une pression de consigne $P_c$ et la pression mesurée par le capteur 4. Cette pression mesurée est transmise, d'une part à part à un transformateur analogique digital AD destiné à transmettre la valeur mesurée à un affichage A et, d'autre part, à une entrée d'un amplificateur différentiel $A_2$ dont l'autre entrée est connectée à une valeur donnant la limite inférieure de pression tolérée $P_i$.

Le moteur $M_7$ est associé à une dynamo tachymétrique T qui est connectée à l'entrée d'un différentiateur $D_2$ dont l'autre entrée est reliée sélectivement par un commutateur C à deux valeurs de consignes du débit $CD_1$ respectivement $CD_2$. La sortie de ce différentiateur $D_2$ est connectée à l'entrée d'un amplificateur $A_3$ dont la sortie est reliée au moteur $M_7$ par l'intermédiaire d'un interrupteur $I_2$.

La figure 3 illustre la commande du dispositif qui comporte deux étages de commutation, l'un comprenant un interrupteur $I_3$ associé à deux interrupteurs $I_4$ et $I_5$ du second étage qui comporte encore un troisième interrupteur $I_6$. Les interrupteurs $I_4$ et $I_5$ sont montés en série avec deux relais respectifs $R_G$ et $R_D$ shuntés par deux diodes $Di_1$ et $Di_2$. Chacun de ces relais R commande un interrupteur $I_G$ respectivement $I_D$ de mise en marche de l'outil de coupe chirurgical pour faire tourner cet outil sélectivement à gauche ou à droite. Ces interrupteurs $I_4$ et $I_5$ sont encore connectés à un relais R connecté en série avec l'interrupteur $I_6$, par des diodes $Di_3$, $Di_4$ et $Di_5$. Ce relais R sert à actionner le commutateur C (fig. 2) pour amener le différentiateur en connection avec les consignes de débit 1 ou 2 ($CD_1$, $CD_2$). L'interrupteur $I_3$ est encore connecté à un relais $R_V$ de commande de la vanne trois voies 8 par les diodes $Di_3$ ou $Di_4$.

Grâce à cette disposition, les interrupteurs $I_4$ ou $I_5$ fermés commandent toujours le commutateur C (fig. 2) sur la position $CD_2$ correspondant au régime de rinçage élevé et le relais $R_V$ qui commute la vanne trois voies 8 sur le conduit d'aspiration auxiliaire, c'est-à-dire à travers le conduit recevant l'outil de coupe, même si celui-ci a été enlevé. L'interrupteur $I_3$ étant ouvert, ni le relais $R_G$ ni le relais $R_D$ ne sont alimentés, et les interrupteurs $I_G$ et $I_D$ restent ouverts, l'outil de coupe ne fonctionne donc pas. Ceci permet, comme on l'a dit précédemment de diriger localement l'aspiration à régime élevé à travers le conduit auxiliaire.

Si maintenant le chirurgien veut continuer la résection endoscopique, il branche à nouveau l'outil de coupe au conduit auxiliaire 6 et il ferme l'interrupteur $I_3$. Celui-ci a pour effet de faire passer le courant dans le relais $R_G$ ou $R_D$ suivant que l'interrupteur $I_4$ respectivement $I_5$ est fermé. Outre l'aspiration à régime élevé commandée par le relais R et la commutation de la vanne trois voies sur le conduit auxiliaire 6, la fermeture des interrupteurs $I_4$ et $I_5$ provoque la fermeture des interrupteurs $I_G$ respectivement $I_D$ par l'intermédiaire des relais $R_G$ respectivement $R_D$. Quant à l'interrupteur $I_6$, que l'interrupteur $I_3$ soit fermé ou non, il n'actionne que le relais R et commande donc l'aspiration à régime élevé à travers le conduit normal 5.

Généralement, pendant une intervention, le chirurgien fait fermer l'interrupteur $I_3$. Ensuite, par l'intermédiaire d'une pédale à trois commandes, il peut par le même interrupteur obtenir sélectivement la mise en marche de l'outil de coupe avec sens de rotation dextre ou senestre et l'aspiration simultanée à régime élevé à travers cet outil ou encore l'aspiration à régime élevé à travers le conduit normal 5. Si aucun interrupteur $I_4$, $I_5$ ou $I_6$ n'est fermé, l'aspiration est automatiquement commutée sur la consigne de débit $CD_1$ par le commutateur C (fig. 2).

Au cas où la pression viendrait à chuter au-dessous d'un seuil limite $P_i$, la sortie de l'amplificateur différentiel $A_2$ ouvre les interrupteurs $I_1$ et $I_2$ arrêtant a la fois les pompes 2 et 7. Il s'agit là d'une sécurité, notamment au cas où la source de liquide physiologique est tarie.

Les trois commandes des interrupteurs $I_4$, $I_5$ et $I_6$ se trouvent sur une même pédale ce qui permet au chirurgien d'effectuer, sans aide et sans utiliser ses mains, la commande de ces interrupteurs et des éléments qui leur sont associés, c'est-à-dire, l'électrovanne 8, l'organe de résection endoscopique et le commutateur C.

## Revendications

1. Circuit électrique de commande d'un dispositif pour faire circuler un liquide physiologique à débit et pression déterminés à travers une cavité du corps, en vue d'une résection et/ou d'un examen endoscopique, dans lequel, d'une part, un conduit d'alimentation est destiné à relier une source de ce liquide (1) à ladite cavité (G) par l'intermédiaire d'une pompe d'alimentation (2) et d'un capteur de pression (4) associé à un circuit d'asservissement de cette pompe en fonction de la pression mesurée par la capteur (4) et, d'autre part, au moins un conduit de sortie (5,6) destiné à évacuer le liquide de cette cavité (G) par l'intermédiaire d'une source d'aspiration (7) commandée par un circuit de contrôle ($D_2$, $A_3$, $I_2$, $M_7$, T), ce circuit de commande comprenant des éléments de consigne ($CD_1$, $CD_2$) pour fixer des valeurs respectives du débit de cette source d'aspiration (7) et des moyens (R, C) pour commuter le circuit de contrôle de cette source sur l'un ou l'autre de ces éléments de consigne ($CD_1$, $CD_2$), caractérisé par le fait qu'il comporte au moins un organe d'actionnement ($I_4$, $I_5$) relié d'une part auxdits moyens (R, C) pour commuter le circuit de contrôle de cette source d'aspiration (7) sur l'un desdits éléments de consigne ($CD_1$, $CD_2$) et, d'autre part, à un élément ($R_G$, $R_D$) d'actionnement d'un interrupteur auxiliaire ($I_G$, $I_D$) de commande d'un organe de résection endoscopique.

2. Circuit selon la revendication 1, caractérisé par le fait qu'il comporte deux organes d'actionnement ($I_4$, $I_5$) reliés d'une part, auxdits moyens (R, C) pour commuter le circuit de contrôle de la source d'aspiration (7) sur l'un desdits éléments de consigne ($CD_1$, $CD_2$) et, d'autre part, à deux éléments ($R_G$, $R_D$) d'actionnement de deux interrupteurs auxiliaires respectifs ($I_G$, $I_D$) de commande de l'organe de résection endoscopique, l'actionnement de chacun de ces deux interrupteurs auxiliaires déterminant un sens de rotation de l'organe de résection.

3. Circuit selon la revendication 2, de commande d'un dispositif pour faire circuler un liquide physiologique à travers une cavité (G) du corps dans lequel deux conduits de sortie (5,6) sont destinés à évacuer le liquide de cette cavité par l'intermédiaire de ladite source d'aspiration, une électrovanne (8) mettant l'un ou l'autre de ces conduits de sortie en communication avec la source d'aspiration, caractérisé par le fait qu'il comporte un troisième organe d'actionnement ($I_6$) relié uniquement auxdits moyens (R, C) pour commuter le circuit de contrôle de la source d'aspiration sur l'un ou l'autre des éléments de consigne, les deux autres organes d'actionnement étant en outre reliés à un élément de commande ($R_V$) de ladite électrovanne (8) pour commuter la source d'aspiration sur un conduit de sortie destiné à recevoir ledit organe de résection endoscopique.

4. Circuit selon l'une des revendications 2 ou 3, caractérisé par le fait que lesdits deux premiers organes d'actionnement ($I_4$, $I_5$) et les éléments ($R_G$, $R_D$) pour les relier à un interrupteur auxiliaire ($I_G$,$I_D$) de commande de l'organe de résection sont montés en série avec un second interrupteur ($I_3$).

**Patentansprüche**

1. Elektrischer Schaltkreis für eine Einrichtung zum Durchströmen eines Körperhohlraums mit einer physiologischen Flüssigkeit mit bestimmtem Durchsatz und Druck zum Zweck einer Resektion und/oder einer endoskopischen Untersuchung, die einerseits eine Speiseleitung zur Verbindung einer diese Flüssigkeit liefernden Quelle (1) mit dem Hohlraum (G) über eine Speisepumpe (2) und einen Druckfühler (4), dem eine Schaltung zur Steuerung der Pumpe in Abhängigkeit von dem von dem Druckfühler (4) gemessenen Druck zugeordnet ist, und andererseits wenigstens eine Ausgangsleitung (5, 6) zur Abfuhr der Flüssigkeit aus dem Hohlraum (G) über eine Saugeinrichtung (7) aufweist, die durch eine Steuerschaltung ($D_2$, $A_3$, $I_2$, $M_7$, T) gesteuert ist, wobei der Schaltkreis Sollwertelemente ($CD_1$, $CD_2$) zur Festlegung der jeweiligen Werte des Durchsatzes der Saugeinrichtung (7) und Einrichtungen (R, C) zur Umschaltung der Steuerschaltung der Saugeinrichtung auf das eine oder das andere Sollwertelement ($CD_1$, $CD_2$) besitzt, gekennzeichnet durch wenigstens ein Betätigungsorgan ($I_4$, $I_5$), das einerseits mit den Einrichtungen (R, C) zur Umschaltung der Steuerschaltung der Saugeinrichtung (7) auf eines der Sollwertelemente ($CD_1$, $CD_2$) und andererseits mit einem Element ($R_G$, $R_D$) zur Betätigung eines Hilfsschalters ($I_G$, $I_D$) zur Steuerung eines Organs zur endoskopischen Resektion verbunden ist.

2. Schaltkreis nach Anspruch 1, gekennzeichnet durch zwei Betätigungsorgane ($I_4$, $I_5$), die einerseits mit den Einrichtungen (R, C) zur Umschaltung der Steuerschaltung der Saugeinrichtung (7) auf eines der Sollwertelemente ($CD_1$, $CD_2$) und andererseits mit zwei Elementen ($R_G$, $R_D$) zur Betätigung von zwei Hilfsschaltern ($I_G$, $I_D$) zur Steuereung des Organs zur endoskopischen Resektion verbunden sind, wobei die Betätigung jedes der beiden Hilfsschalter eine Drehrichtung des Resektionsorgans bestimmt.

3. Schaltkreis nach Anspruch 2 für eine Einrichtung zum Durchströmen eines Körperhohlraums (G) mit einer physiologischen Flüssigkeit, die zwei Ausgangsleitungen (5, 16) zur Abfuhr der Flüssigkeit aus dem Körperhohlraum über die Saugeinrichtung besitzt, wobei ein Magnetventil (8) die eine oder die andere Ausgangsleitung mit der Saugeinrichtung in Verbindung setzt, gekennzeichnet durch ein drittes Betätigungsorgan ($I_6$), das nur mit den Einrichtungen (R, C) zur Umschaltung der Steuerschaltung der Saugeinrichtung auf das eine oder das andere Sollwertelement verbunden ist, wobei die beiden anderen Betätigungsorgane außerdem mit einem Steuerelement ($R_V$) des Magnetventils (8) verbunden sind, um die Saugeinrichtung auf eine Ausgangsleitung umzuschalten, die zur Aufnahme des Organs zur endoskopischen Resektion bestimmt ist.

4. Schaltkreis nach einem der Ansprüche 2 oder 3, dadurch gekennzeichnet, daß die ersten Betätigungsorgane ($I_4$, $I_5$) und die Elemente ($R_G$, $R_D$) zu ihrer Verbindung mit einem Hilfsschalter ($I_G$, $I_D$) zur Steuerung des Resektionsorgans mit einem zweiten Schalter ($I_3$) in Reihe geschaltet sind.

**Claims**

1. Electrical control circuit for a device for circulating a physiological liquid of predetermined flow rate and pressure through a cavity of the body, with a view to resection and/or endoscopic examination, in which, on the one hand, a supply channel is intended to connect a source of this liquid (1) to the said cavity (G) by way of a supply pump (2) and of a pressure sensor (4) which is associated with an automatic control circuit of this pump as a function of the pressure measured by the sensor (4) and, on the other hand, at least one outlet channel (5, 6) intended to discharge the liquid from this cavity (G) by way of a sunction source (7) controlled by a control circuit ($D_2$, $A_3$, $I_2$, $M_7$, T), this control circuit comprising reference value elements ($CD_1$, $CD_2$) for fixing respective values of the flow rate of this suction source (7) and means (R, C) for switching the control circuit from this source over to one or the other of these reference value elements ($CD_1$, $CD_2$), characterized in that it has at least one actuating device ($I_4$, $I_5$) connected on the one hand to the said means (R, C) for switching the control circuit from this suction source (7) over to one of the said reference value elements ($CD_1$, $CD_2$) and on the other hand to an actuating element ($R_G$, $R_D$) of an auxiliary switch ($I_G$, $I_D$) for controlling an endoscopic resection device.

2. Circuit according to Claim 1, characterized in that it has two actuating devices ($I_4$, $I_5$) connected on the one hand to the said means (R, C) for switching the control circuit from the suction source (7) over to one of the said reference value elements ($CD_1$, $CD_2$) and on the other hand to two actuating elements ($R_G$, $R_D$) of two respective auxiliary switches ($I_G$, $I_D$) for controlling the endoscopic resection device, the actuation of each of these two auxiliary switches determining a direction of rotation of the resection device.

3. Circuit according to Claim 2, for controlling a device for circulating a physiological liquid through a cavity (G) of the body in which two outlet channels (5, 6) are intended for discharging the liquid from this cavity by way of the said suction source, an electrovalve (8) allowing one or the other of these channels to communicate with the suction source, characterized in that it has a third actuating device ($I_6$) connected solely to the said means (R, C) for switching the control circuit from the suction source over to one or the other of the reference value elements, the other two actuating devices being further connected to a control element ($R_V$) of the said electrovalve (8) for switching the suction source onto an outlet channel intended to receive the said endoscopic resection device.

4. Circuit according to either of Claims 2 or 3, characterized in that the said first two actuating devices ($I_4$, $I_5$) and the elements ($R_G$, $R_D$) for connecting them to an auxiliary switch ($I_G$, $I_D$) for controlling the resection device are mounted in series with a second switch ($I_3$).

FIG. 1

FIG. 2

FIG. 3

6